# EUROPÄISCHE PATENTSCHRIFT

(11) **EP 2 114 907 B1**
(45) Veröffentlichungstag und Bekanntmachung des Hinweises auf die Patenterteilung: **24.09.2014**
(21) Anmeldenummer: 08707434.0
(22) Anmeldetag: 31.01.2008
(51) Int. Cl.: C07D 295/18, A61K 8/49

(54) **PIPERAZINDERIVATE IN DIOLHALTIGEN KOSMETISCHEN ZUBEREITUNGEN**
PIPERAZINE DERIVATIVES IN COSMETIC PREPARATIONS CONTAINING DIOLS
DÉRIVÉS DE PIPÉRAZINE DANS DES PRÉPARATIONS COSMÉTIQUES CONTENANT DES DIOLS

(30) Priorität: 01.02.2007 DE 102007005334
(43) Veröffentlichungstag der Anmeldung: 11.11.2009
(73) Patentinhaber: Beiersdorf AG, 20253 Hamburg (DE)
(72) Erfinder: REINECKE, Myriam, 22529 Hamburg (DE); BLATT, Thomas, 22880 Wedel (DE); WOLBER, Rainer, 22397 Hamburg (DE); SMUDA, Christoph, 25474 Bönningstedt (DE); CLAUSEN, Andreas, 20146 Hamburg (DE); MUNDT, Claudia, 8037 Zürich (CH)
(86) Internationale Anmeldenummer: PCT/EP2008/000745
(87) Internationale Veröffentlichungsnummer: WO 2008/092672

(56) Entgegenhaltungen:
- WO-A-2004/052837
- "Use of amino hydroxy benzophenone derivatives in sunscreen preparations" IP.COM JOURNAL, IP.COM INC., WEST HENRIETTA, NY, US, 24. August 2006 (2006-08-24), XP013115567 ISSN: 1533-0001
- "Suncare compositions using amino hydroxy benzophenone derivatives" IP.COM JOURNAL, IP.COM INC., WEST HENRIETTA, NY, US, 3. Oktober 2007 (2007-10-03), XP013122291 ISSN: 1533-0001

## Beschreibung

Die vorliegende Erfindung betrifft eine Kombination aus UV-Licht filternden Piperazinderivaten mit 1,3-Alkandiolen.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfitersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen:

Kosmetische Zubereitungen, die wie Sonnenschutzzubereitungen auf die Haut aufgetragen werden, kommen regelmäßig (beabsichtigt oder unbeabsichtigt) mit Kleidungsstücken in Kontakt, an denen sie (z.B. als "Abrieb" oder weil sie von den Faserstoffen "aufgesaugt" werden) teilweise haften bleiben. Insbesondere wenn die kosmetischen Zubereitungen eine leichte Eigenfarbe aufweisen oder einen gewissen Anteil an lipophilen Komponenten enthalten (wie es bei nahezu jeder Emulsion oder Dispersion der Fall ist) führt dies zu einer optisch wahrnehmbaren Verunreinigung (z.B. Verfärbung) des Kleidungsstückes. Besonders auffällig ist dieser Effekt bei weißen Kleidungsstücken. Wenn beispielsweise in Vorbereitung auf ein Sonnenrad schon "zu Hause" das Sonnenschutzmittel auf die Haut aufgetragen wird und auf dem Weg zum "Badeort eine leichte Sommerbekleidung (T-Shirts, Hemden, Röcke, Shorts) getragen wird, kommen diese Textilien unmittelbar mit der frisch applizierten Lichtschutzzubereitung in Kontakt und nehmen mehr oder weniger große Mengen davon auf. Ebenso häufig kommt es nach Beendigung des Sonnenbades zu einem Transfer von "Kosmetik-Rückständen" (Überresten des Sonnenschutzmittels) auf die dann vom Sonnenanbeter wieder angezogene Kleidung. Besonders intensiv ist regelmäßig der Kontakt von Badebekleidung (z.B. Bikinis, Badehosen, Badeanzügen, Handtüchern, Badelaken) und Sonnenschutzmitteln.

Nachteilig am Stande der Technik ist dabei insbesondere der Umstand, dass die einmal von den Textilien aufgenommenen Kosmetika (z.B. Sonnenschutzmittel) sich nur relativ schwer wieder auswaschen lassen. Regelmäßig bleiben mehr oder weniger große Reste des Kosmetikums auch nach einem Waschgang noch auf der Kleidung zurück. Nach dem Stand der Technik bleibt dem Verbraucher häufig nur die Möglichkeit die Kleidungsstücke wiederholt zu waschen, bei höherer Temperatur zu waschen und/oder größere Mengen an Waschmittel aufzuwenden. All diese Maßnahmen führen zu einer erhöhten Umweltbelastung und zu einem erhöhten Verschleiß der Textilien, die dadurch häufiger ersetzt werden müssen.

Es war daher die Aufgabe der vorliegenden Erfindung diesen Nachteil des Standes der Technik zu beseitigen. Insbesondere war es die Aufgabe der vorliegenden Erfindung, kosmetische Zubereitungen (insbesondere Sonnenschutzmittel) zu entwickeln, welche die Haut, Haare und Nägel zuverlässig vor den negativen Auswirkungen des Sonnenlichtes schützen und sich dabei besonders leicht und für das menschliche Auge sichtbar aus den mit diesen Zubereitungen verunreinigten Kleidungsstücken (inklusive Halstücher) wieder auswaschen lassen.

Überraschend gelöst werden die Aufgaben durch eine kosmetische Zubereitung enthaltend
a) 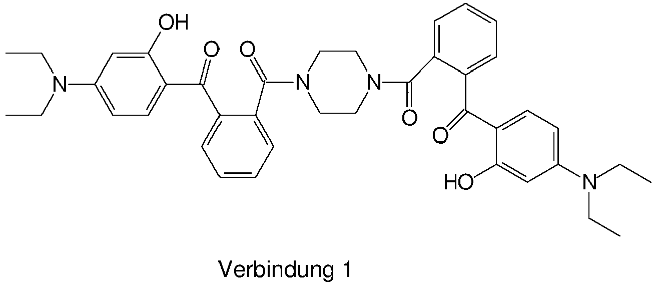
b) 2-Methylpropan-1,3-diol.

Zwar beschreibt die Internet-Veröffentlichung IPCOM000134143 UV-Filter auf der Basis von Piperazinderivaten in kosmetischen Sonnenschutzmitteln, doch konnte diese Schrift nicht den Weg zur vorliegenden Erfindung weisen. Darüber hinaus kennt der Fachmann die Internet-Veröffentlichung IPCOM000139425 sowie die WO 2004/052837, die ebenfalls nicht den Weg zur vorliegenden Erfindung weisen konnten.

Die erfindungsgemäßen Zubereitungen zeichnen sich durch einen exzellenten UV-A-Schutz aus. Darüber hinaus sind die Zubereitungen überraschend photostabil, d.h. dass sowohl die UV-Lichtschutzfilter als auch die sonstigen Bestandteile gegenüber Licht stabiler sind, als man es erwartet hätte. Nicht zuletzt zeigen die erfindungsgemäßen Zubereitungen eine bessere Resistenz gegen Hefen, die ansonsten zu einem Verderben der Zubereitung führen.

Die erfindungsgemäßen Zubereitungen zeigen einen überraschend breiten UV-Absorptionsbereich, hohe spezifische Absorption in diesem Bereich, Photo- und Thermostabilität, Hautverträglichkeit (keine reizenden und toxischen Wirkungen auf der Haut), gutes Hautgefühl und gute Haftung auf der Haut, Wasserfestigkeit sowie gute Verträglichkeit mit anderen kosmetischen Substanzen.

Darüber hinaus zeigen die Zubereitungen hervorragende kosmetische Eigenschaften, da die Hautbefeuchtung der Zubereitung durch die erfindungsgemäße Kombination verbessert wird und die Klebrigkeit auf der Haut reduziert ist.

Es ist erfindungsgemäß vorteilhaft, wenn die Zubereitung Verbindung 1 in einer Konzentration von 0,25 bis 10,0 Gewichts-% und bevorzugt in einer Konzentration von 0,5 bis 5,0 Gewichts-%, jeweils bezogen auf das Gesamtgewicht der Zubereitung, enthält.

Es ist erfindungsgemäß vorteilhaft, Verbindung 1 in Form einer wässrigen Dispersion zuzusetzen. Derartige Dispersionen enthalten Verbindung 1 in der Regel in einem Gehalt von 45 bis 50 Gewichts-%, bezogen auf das Gesamtgewicht der Dispersion. Als Dispergierhilfen enthalten solche Dispersionen in der Regel die üblichen Dispergiermittel, beispielsweise Plantacare 2000 UP (Cognis), Amphisol K (DSM), Texapon K14S Spezial (Cognis)Rhodicare S (C.H. Erbslöh KG), Silfoam SE2 (Wacker), Butylenglycol, Zitronensäure, NaOH.

Es ist erfindungsgemäß vorteilhaft, wenn die Zubereitung 1,3-Alkandiole in einer Konzentration von 0,2 bis 5 Gewichts-% und erfindungsgemäß bevorzugt in einer Konzentration von 0,5 bis 2,0 Gewichts-%, jeweils bezogen auf das Gesamtgewicht der Zubereitung, enthält.

Erfindungsgemäß vorteilhafte Ausführungsformen der vorliegenden Erfindung sind dadurch gekennzeichnet, dass die Zubereitung einen oder mehrere weitere UV-Filter enthält, gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, 4-(tert.-Butyl)-4'-methoxydibenzoylmethan; Homomenthylsalicylat; 2-Ethylhexyl-2-hydroxybenzoat; 2-Ethylhexyl-2-cyano-3,3-diphenylacrylat; Dimethicodiethylbenzalmalonat; 3-(4-(2,2-bis Ethoxycarbonylvinyl)-phenoxy)propenyl)-methoxysiloxan / Dimethylsiloxan - Copolymer; 2,4-Bis-{[4-(2-ethyl-hexyloxy)-2-hydroxy]-phenyl}-6-(4-methoxyphenyl)-1,3,5-triazin (INCI :Bis-Ethylhexyloxyphenol Methoxyphenyl Triazin); Dioctylbutylamidotriazon (INCI: Diethylhexyl-Butamidotriazone); 2,4-bis-[5-1 (dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'--ethyl-1'-hexyloxy)]-1,3,5-triazin (INCI: Ethylhexyl Trazone); 2,4,6-Tribiphenyl-4-yl-1,3,5-triazin; Merocyanine; Titiandioxid; Zinkoxid in einer Konzentration von 0,01 bis 40 Gewichts-% bezogen auf das Gesamtgewicht der Zubereitung.

Vorteilhafte Ausführungsformen der vorliegenden Erfindung sind auch dadurch gekennzeichnet, dass die Zubereitung als weitere Inhaltsstoffe eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, Polydocanol, natürliche und/oder synthetische Isoflavonoide, Flavonoide, Kreatin, Kreatinin, Taurin, ß-Alanin, Tocopherylacetat, Harnstoff; Hyaluronsäure; Dihydroxyaceton; 8-Hexadecen-1,16-dicarbonsäure und/oder Licochalcon A enthält. Derartige Wirkstoffe können in Einzelkonzentrationen von 0,001-20 Gewichts-%, bezogen auf das Gesamtgewicht der Zubereitung, in dieser enthalten sein.

Es ist erfindungsgemäß vorteilhaft, wenn die Zubereitung in Form einer Emulsion, einer Dispersion, eines Puders, eines Gels oder eines Öls vorliegt. Erfindungsgemäß besonders bevorzugt ist die Ausführungsform der Emulsion.

Es ist erfindungsgemäß besonders bevorzugt, wenn die erfindungsgemäße Zubereitung in Form einer O/W-Emulsion vorliegt.

Liegt die erfindungsgemäße Zubereitung in Form einer O/W-Emulsion vor, so ist sie erfindungsgemäß bevorzugt dadurch gekennzeichnet, dass die Zubereitung einen oder mehrere O/W-Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Ceteareth-20, PEG-40 Stearat, Natriumcetearylsulfat enthält. Ferner ist es vorteilhaft im Sinne der vorliegenden Erfindung Cetearylalkohol in Kombination mit PEG-40 hydriertes Rizinusöl, Natriumcetearylsulfat und Glycerylstearat. Ausserdem ist es erfindungsgemäß vorteilhaft Kaliumcetylphosphat als Emulgator einzusetzen.

Diese erfindungsgemäßen O/W-Emulgatoren können erfindungsgemäß vorteilhaft in einer Konzentration von 0,001 bis 10 Gewichts-% und bevorzugt in einer Konzentration von 0,1 bis 7 Gewichts-%, bezogen auf das Gesamtgewicht der Zubereitung in dieser enthalten sein.

In einer weiteren erfindungsgemäß besonders bevorzugten Ausführungsform liegt die erfindungsgemäße Zubereitung in Form einer W/O-Emulsion vor.

In dieser Ausführungsform ist es erfindungsgemäß besonders bevorzugt, wenn die Zubereitung einen oder mehrere W/O-Emulgatoren gewählt aus der Gruppe der Verbindungen Polyglyceryl-2-dipolyhydroxystearat, PEG-30 Dipolyhydroxystearat, Cetyl Dimethicon Copolyol, Polyglyceryl-3 Diisostearat enthält.

Diese erfindungsgemäßen W/O-Emulgatoren können erfindungsgemäß vorteilhaft in einer Konzentration von 0,1 bis 10 Gewichts-% und bevorzugt in einer Konzentration von 0,2 bis 7 Gewichts-%, bezogen auf das Gesamtgewicht der Zubereitung in dieser enthalten sein.

Es ist vorteilhaft im Sinne der vorliegenden Erfindung, wenn die Zubereitung ein oder mehrere Verbindungen gewählt aus der Gruppe der Parabene, Phenoxyethanol, Ethylhexylglycerin, Butylenglycol, Propylenglycol enthält. Derartige Inhaltsstoffe können bevorzugt in einer Einzelkonzentration von 0,001 bis 10 Gewichts-% in der Zubereitung enthalten sein.

Besonders vorteilhafte Ausführungsformen der vorliegenden Erfindung erhält man auch dadurch dass die Zubereitung als weitere Inhaltsstoffe eine oder mehrere Verbindungen gewählt aus der Gruppe der Verbindungen eine oder mehrere der Verbindungen 2 bis 9 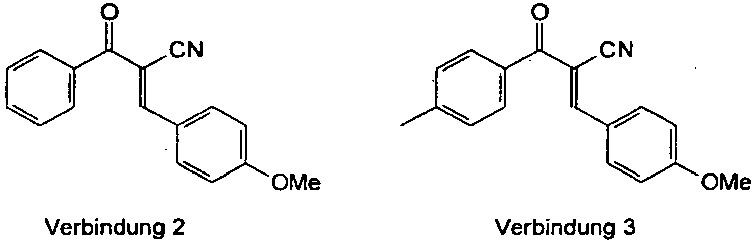 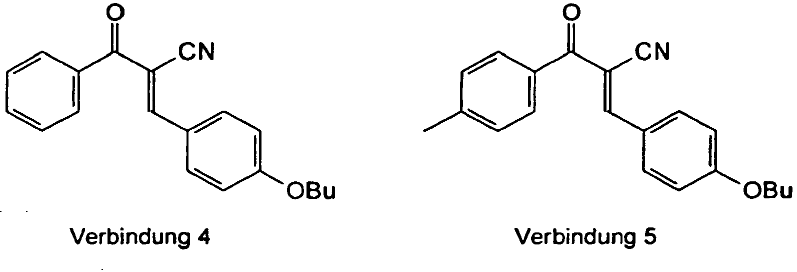 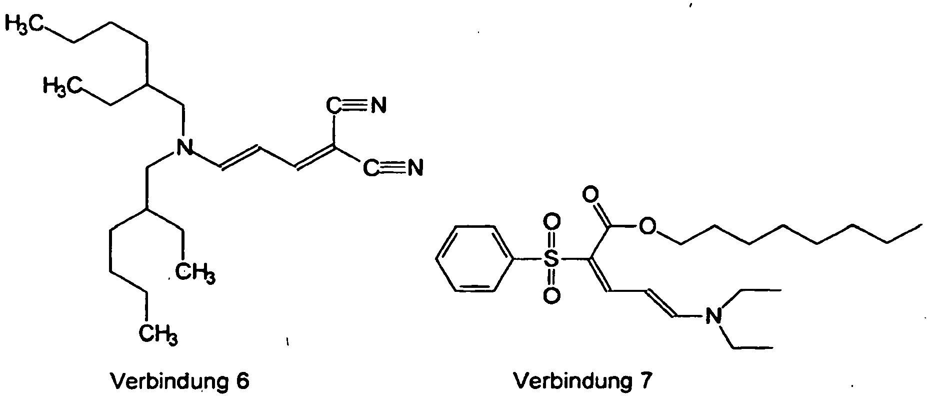 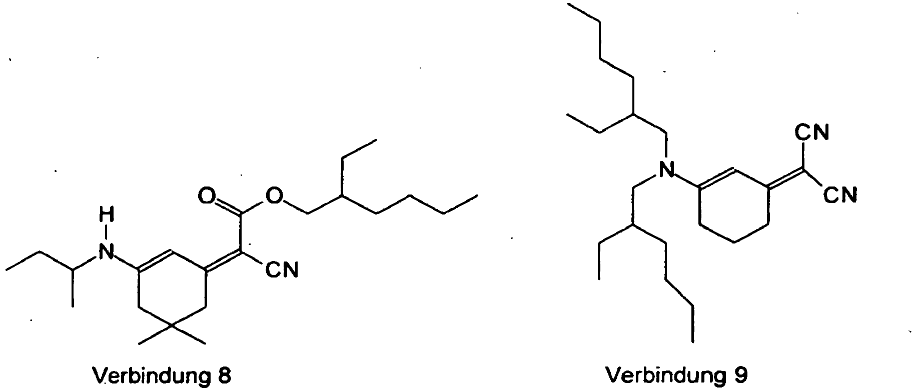 enthält. Derartige Verbindungen liegen erfindungsgemäß vorteilhaft in Einzelkonzentrationen von 0,1 bis 15 Gewichts-%, bezogen auf das Gesamtgewicht der Zubereitung in dieser vor.

Es ist erfindungsgemäß vorteilhaft, wenn die erfindungsgemäße Zubereitung frei ist von p-Methylbenzylidencampher.

Die Pigmente (Titandioxid, Zinkoxid) können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Die Pigmente (Titandioxid, Zinkoxid) können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), Bariumsulfat (BaSO₄) oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Die erfindungsgemäßen Zubereitungen können ferner vorteilhaft auch Selbstbräunungssubstanzen enthalten, wie beispielsweise Dihydroxyaceton und/oder Melaninderivate in Konzentrationen von 1 Gew.-% bis zu 10 Gew.-%, bezogen auf das Gesamtgewicht der Zubereitung.

Ferner vorteilhaft können die erfindungsgemäßen Zubereitungen auch Repellentien zum Schutz vor Mücken, Zecken und Spinnen und dergleichen enthalten. Vorteilhaft sind z. B. N,N-Diethyl-3-methylbenzamid (Handelsbezeichnung: Meta-delphene, "DEET"), Dimethylphtalat (Handelsbezeichnung: Palatinol M, DMP), 1-Piperidincarbonsäure-2-(2-hydroxyethyl)-1-methylpropylester sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Es ist vorteilhaft im Sinne der vorliegenden Erfindung, wenn die erfindungsgemäße Zubereitung ein oder mehrere Feuchthaltemittel in einer Gesamtkonzentration von 0,1 bis 20 Gewichts-% und bevorzugt in einer Gesamtkonzentration von 0,5 bis 10 Gewichts-%, jeweils bezogen auf das Gesamtgewicht der Zubereitung, enthält.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9) und /oder Talkum.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, 2-Methylpropan-1,3-diol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß vorteilhafte Verdicker sind solche mit der INCI-Bezeichnung Acrylates/C10-30 Alkyl Acrylate Crosspolymer (z. B. Pemulen TR 1, Pemulen TR 2, Carbopol 1328 von der Fa. NOVEON) sowie Aristoflex AVC (INCI: Ammonium AcryloyldimethyltaurateNP Copolymer).

Erfindungsgemäß vorteilhaft enthält die erfindungsgemäße Zubereitung Filmbildner. Filmbildner im Sinne der vorliegenden Erfindung sind Stoffe unterschiedlicher Zusammensetzung, die durch die folgende Eigenschaft charakterisiert sind: Löst man einen Filmbildner in Wasser oder anderen geeigneten Lösungsmitteln und trägt die Lösung dann auf die Haut auf, so bildet er nach dem Verdunsten des Lösemittels einen Film aus, der im wesentlichen dazu dient, die Lichtfilter auf der Haut zu fixieren und so die Wasserfestigkeit des Produktes zu steigern.

Es ist insbesondere von Vorteil, die Filmbildner aus der Gruppe der Polymere auf Basis von Polyvinylpyrrolidon (PVP) 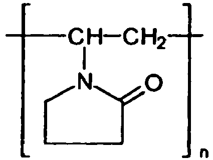 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Ebenfalls vorteilhaft sind weitere Polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000. Weitere geeignete Polymere sind z.B. Polyacrylamide (Seppigel 305), Polyvinylalkohole, PVP, PVP / VA Copolymere, Polyglycole, Acrylat/Octylacralymid Copolymer (Dermacryl 79). Ebenfalls vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

Die Ölphase der erfindungsgemäßen Zubereitung wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB*) und/oder Diethylhexylnaphthalat (*Hallbrite TQ oder Corapan TQ von Symrise*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Die erfindungsgemäßen Zubereitungen können ferner vorteilhaft eine oder mehrere Substanzen aus der folgenden Gruppe der Siloxanelastomere enthalten, beispielsweise, um die Wasserfestigkeit und/oder den Lichtschutzfaktor der Produkte zu steigern:
(a) Siloxanelastomere, welche die Einheiten R₂SiO und RSiO_{1,5} und/oder R₃SiO_{0,5} und/oder SiO₂ enthalten,
   wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
   wobei die verwendeten Mengenateile so gewählt werden, dass die Menge des Wasserstoffes des Organopolysiloxans (1) oder der ungesättigten aliphatischen Gruppen des Organopolysiloxans (2)
   - im Bereich von 1 bis 20 mol-% liegt, wenn das Organopolysiloxan nicht zyklisch ist und
   - im Bereich von 1 bis 50 mol-% liegt, wenn das Organopolysiloxan zyklisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCl-Bezeichnung Dimethicone / Vinyl Dimethicone Crosspolymer, beispielsweise das von DOW CORNING unter der Handelsbezeichnungen DOW CORNING 9506 Powder erhältliche.

Besonders bevorzugt ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische Wirkstoffe enthalten, wobei bevorzugte Wirkstoffe Antioxidantien sind, welche die Haut vor oxidativer Beanspruchung schützen können.

Die kosmetischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhaft im Sinne der vorliegenden Erfindung sind Zubereitungen zur Pflege der Haut: sie können dem kosmetischen Lichtschutz, ferner als Schminkprodukt in der dekorativen Kosmetik dienen.

Entsprechend ihrem Aufbau können kosmetische Zusammensetzungen im Sinne der vorliegenden Erfindung, beispielsweise verwendet werden als Hautschutzcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Es ist auch vorteilhaft im Sinne der vorliegenden Erfindung, kosmetische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamem Schutz der Zubereitungen selbst gegen Verderb dar. Günstig sind ferner kosmetische Zubereitungen, die in der Form eines Sonnenschutzmittels vorliegen.

Erfindungsgemäß ist insbesondere die Verwendung der erfindungsgemäßen Zubereitung zum Schutz vor Hautalterung (insbesondere zum Schutz vor UV-bedingter Hautalterung) sowie als Sonnenschutzmittel.

Erfindungsgemäß vorteilhaft weist die erfindungsgemäße Zubereitung einen pH-Wert von 5 bis 8 auf. Dieser kann durch die herkömmlichen Säuren, Basen und Puffersysteme eingestellt werden.

Die erfindungsgemäße Zubereitung kann beliebige Mischungen von Duft- und Parfümstoffen in den unterschiedlichsten Konzentrationen enthalten.

Zur Anwendung werden die erfindungsgemäßen kosmetischen Zubereitungen in der für Kosmetika üblichen Weise auf die Haut und/oder die Haare in ausreichender Menge aufgebracht.

### Vergleichsversuche

### Vergleichsversuch 1

Um den besonderen UV-Schutz der Sonnenschutzverbindungen (O/W-Emulsionen mit Methylpropandiol) mit der Verbindung 1 zu testen, wurde eine UV-Bestrahlungsstudie durchgeführt, bei der ein Produkt A mit Verbindung 1 (5% als Gewichtsprozent) mit einem Produkt B ohne Verbindung 1 verglichen wurde. Beide Produkte hatten einen Lichtschutzfaktor von 18 und eine UVA-Balance (gemessen nach DIN 67502) von 37. Fünf Probanden (männlich und weiblich) im Alter von 18 bis 60 Jahren, die gut bräunend waren und einen Phototyp II-III hatten, nahmen an der Studie teil. Vor und während der Studie durften keine Sonnenschutzprodukte, keine pflegenden Produkte mit UV-Filtern und keine Selbstbräuner oder Produkte, die Selbstbräuner enthalten, angewandt werden. Ab der vierten Woche vor Studienbeginn durfte kein Solarium besucht werden und natürliche Sonnenbestrahlung musste vermieden werden.

### Lichtschutzfaktor und DIN-Balance der getesteten O/W-Emulsionen:

| Emulsion | Lichtschutzfaktor | DIN-Balance |
|---|---|---|
| A | 18 | 37 |
| B | 18 | 37 |

### Durchführung

Zur Bestimmung der individuellen Lichtempfindlichkeit wurde zu Beginn der Studie bei jedem Probanden eine Lichttreppe gesetzt, die 16 bis 24h nach der Bestrahlung ausgewertet wurde. Die Lichttreppe stellte sicher, dass die Bestrahlungsdosis während der Studie im suberythemalen Bereich lag.

In den folgenden zwei Wochen wurde repetitiv montags bis freitags mit suberythemalen Dosen sonnensimuliertem UV-Lichts für zwanzig Minuten bestrahlt. Die zwei Produkte wurden dafür in einer Schichtdicke von 2 mg/ cm² auf 4 cm x 6 cm großen rechteckigen Arealen, die auf dem Rücken beidseitig der Wirbelsäule angeordnet waren, aufgetragen. Die Produkte wurden nach einem Permutationsschema auf die Felder verteilt. Nach der Produktapplikation wurde fünfzehn Minuten gewartet. Anschließend wurden die Felder mit einer Schablone abgedeckt, die 3cm x 5cm große Felder auf den Produktflächen frei ließ. Diese 15 cm² großen Felder wurden mit einem Oriel IL 1700 (Newport, USA) mit sonnensimuliertem Licht mit einer durchschnittlichen Intensität von 2,1 x 10⁻⁴ W/cm² zwanzig Minuten lang bestrahlt.

### Auswertung

Vor der ersten Bestrahlung, an definierten Zeitpunkten während der Studie, 72h nach der zehnten Bestrahlung und drei Wochen nach der letzten Bestrahlung wurde die Bräunung der Haut makroskopisch und mithilfe eines UV-VIS-Reflexionsspektrometers (Mittelwerte aus jeweils sechs Messungen) mit einem faseroptischem Messkopf (Carl Zeiss, Software Aspect Plus) gemessen und beurteilt.

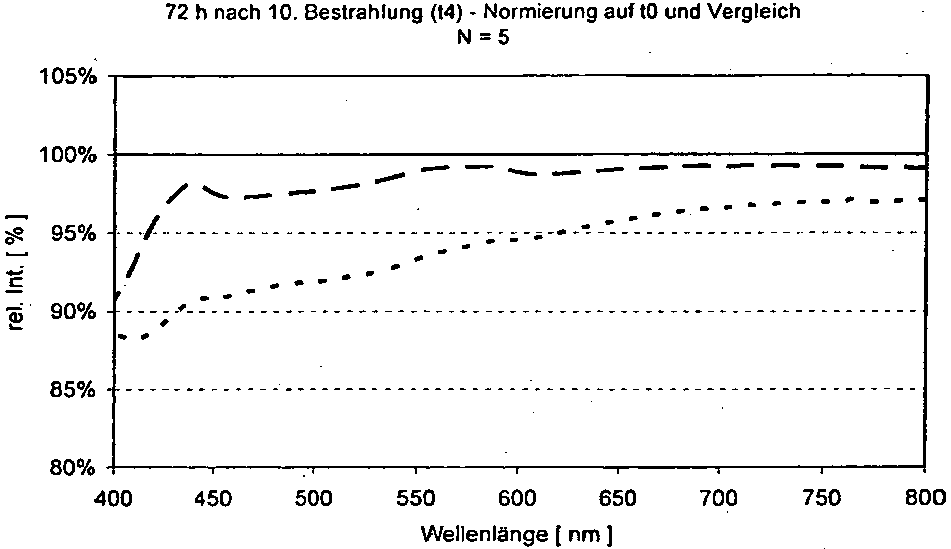

Ergebnisse der Reflexionsspektroskopie: Die Gerade bei 100% repräsentiert die unbehandelten Referenzfelder, die weder eingecremt noch bestrahlt wurden. Die mittlere, gestrichelte Linie zeigt das Produkt A mit Verbindung 1 und die untere Linie zeigt das Produkt B ohne Verbindung 1. Das Produkt A mit Verbindung 1 zeigt einen deutlich besseren Schutz und eine geringere Bräunung als das Produkt B ohne Verbindung 1. Je größer die rel. Int [%] ist, desto besser ist der Schutz und desto geringer ist die Bräunung.

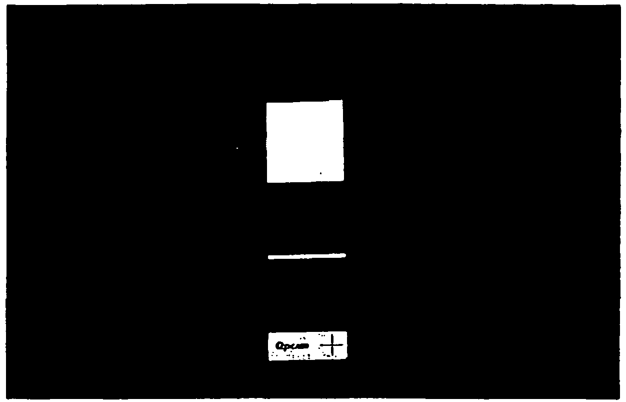

Aufnahme nach der 10. Bestrahlung mit sonnensimuliertem Licht: Das Feld 14 wurde durch das Produkt A mit Verbindung 1 geschützt und ist entsprechend am wenigsten gebräunt. Das Feld 16 ist deutlich stärker gebräunt. Auf diesem Feld wurde das Produkt B ohne Verbindung 1 appliziert.

### Ergebnis

Das Produkt A mit Verbindung 1 hat die Haut aller Probanden deutlich besser geschützt als das Produkt B ohne Verbindung 1, obwohl beide Produkte einen einheitlichen Lichtschutzfaktor von 18 und eine einheitliche UVA-Balance von 37 haben.

### Vergleichsversuch 2

### Rezepturen der verglichenen O/ W-Emulsionen:

| Rohstoff | Muster 3 | Muster 4 | Muster 5 |
|---|---|---|---|
| Wasser | Ad 100 | Ad 100 | Ad 100 |
| C12-15 Alkyl Benzoat | 5.3 | 5.3 | 5.3 |
| Cetearyl Alkohol + PEG-40 Castor Öl + Sodium Cetearyl Sulfat | 2.5 | 2.5 | 2.5 |
| Cetyl Alkohol | 1.5 | 1.5 | 1.5 |
| Dibutyladipat | 3 | 3 | 3 |
| Glycerin | 6.6 | 6.6 | 6.6 |
| Glyceryl Stearat SE | 1 | 1 | 1 |
| Methylpropandiol | 0 | 5 | 5 |
| Parfum | 0.4 | 0.4 | 0.4 |
| Phenethylbenzoat | 5.2 | 5.2 | 5.2 |
| Konservierungsmittel | q.s. | q.s | q.s |
| Sorbitan Stearat | 0.5 | 0.5 | 0.5 |
| Tocopheryl Acetat | 0.5 | 0.5 | 0.5 |
| Trinatrium EDTA | 1 | 1 | 1 |
| VP/ Hexadecen Copolymer | 0.5 | 0.5 | 0.5 |
| Xanthan Gummi | 0.4 | 0.4 | 0.4 |
| Verbindung 1 | 4 | 4 | 0 |

Die Klebrigkeit der O /W-Emulsionen wurde in dem Sandtest untersucht.

### Durchführung des Sandtests:

Die Produkte werden mithilfe einer 10 µl-Eppendorf-Pipette in einer Schichtdicke von 2 mg/ cm² auf den Unterarm des Probanden aufgetragen. Nachdem das Produkt gleichmäßig auf der Haut verteilt worden ist, wird dieses Areal mit Sand bestreut (Schichtdicke: 62,5 mg/ cm²).

Nach 15 s wird dreimal vorsichtig mit der flachen Hand auf den Unterarm geklopft. Der Unterarm wird gedreht, so dass nicht-haftender Sand sich von der Haut löst. Anschließend wird makroskopisch beurteilt, wieviel Sand auf der Haut verblieben ist. Diese restliche Sandmenge ist proportional zu der Klebrigkeit der Emulsion.

### Dokumentation (Foto) des Sandtests:

Muster 3: 4% Verbindung 1
Muster 4: 4% Verbindung 1 + 5% Methylpropandiol 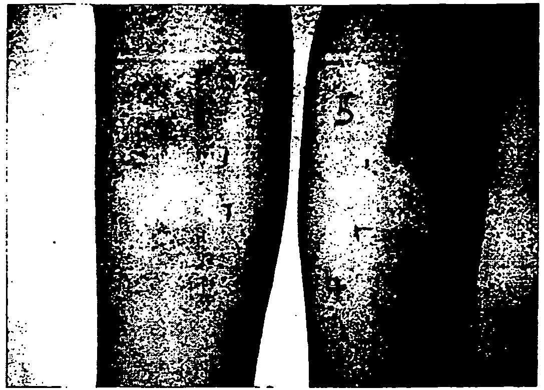
Sandtest: Vergleich der Klebrigkeit der Muster 3, 4 und 5. Das Muster 4 zeigt eine deutlich geringere Klebrigkeit als die Muster 3 und 5.

### Ergebnis des Sandtests:

Bei dem Mustern 3 und 5 ist ein deutlicher Sandrückstand auf der Haut zu erkennen, während bei dem Muster 4 nur wenig Sand auf der Haut verbleibt. Die O/W-Emulsion, die neben 4% Verbindung 1 auch 5% Methylpropandiol enthält (Muster 4), ist folglich deutlich weniger klebrig (bzw. stärker sandabweisend) als die Zubereitungen mit nur einer Komponente.

### Beispiele

Die nachfolgenden Beispiele sollen die vorliegende Erfindung verdeutlichen. Die Angaben beziehen sich stets auf Gewichts-%, sofern nicht andere Angaben gemacht werden.

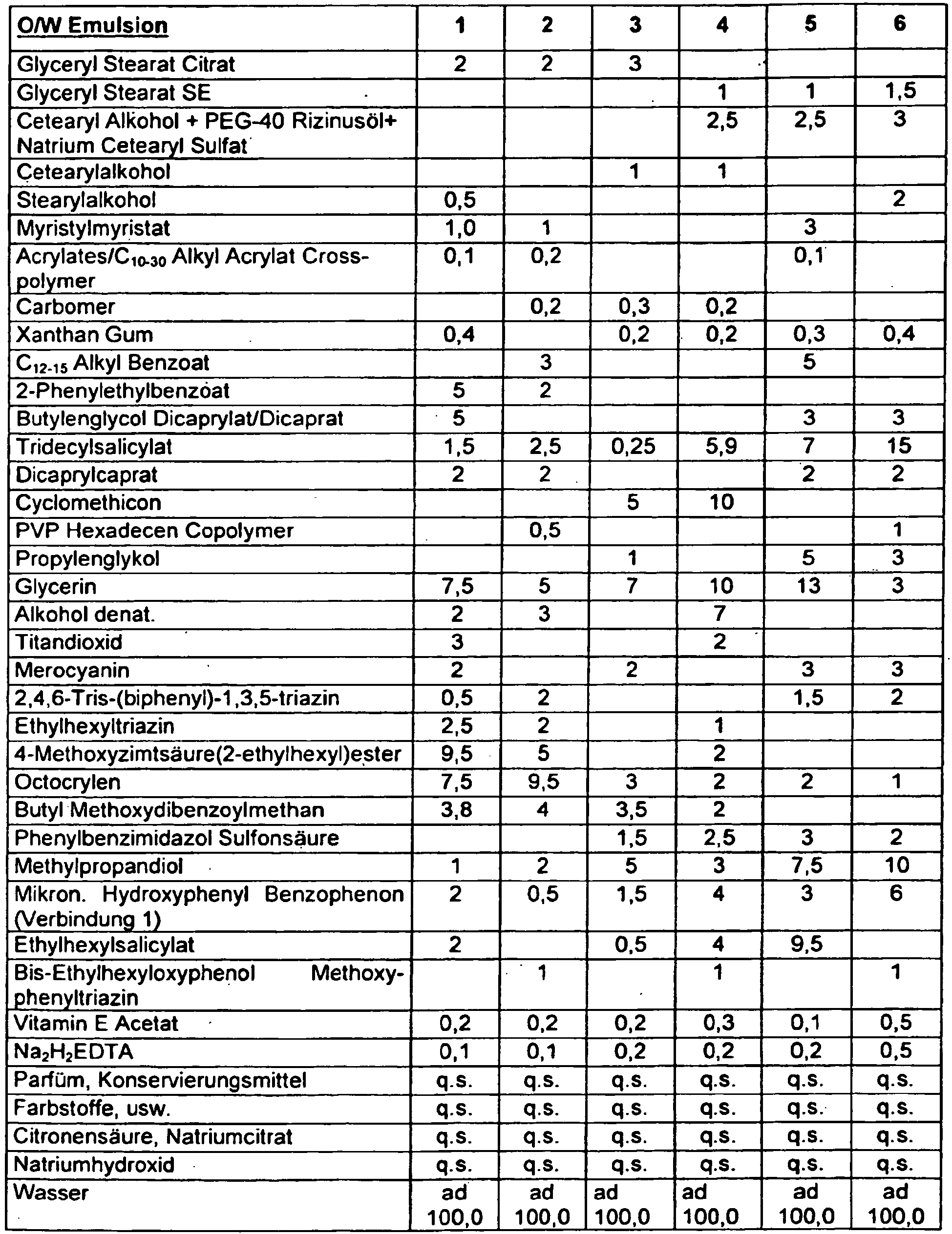

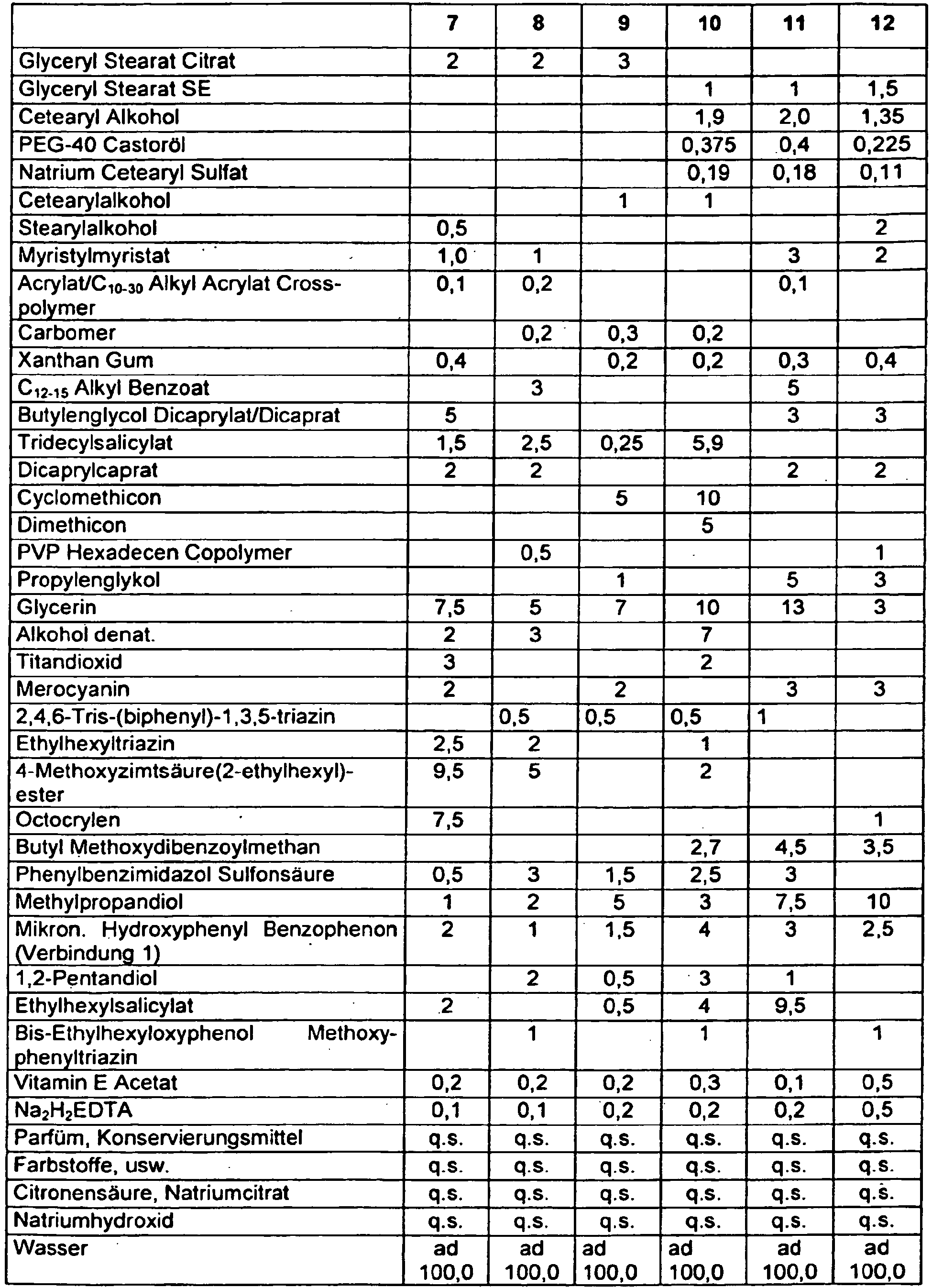

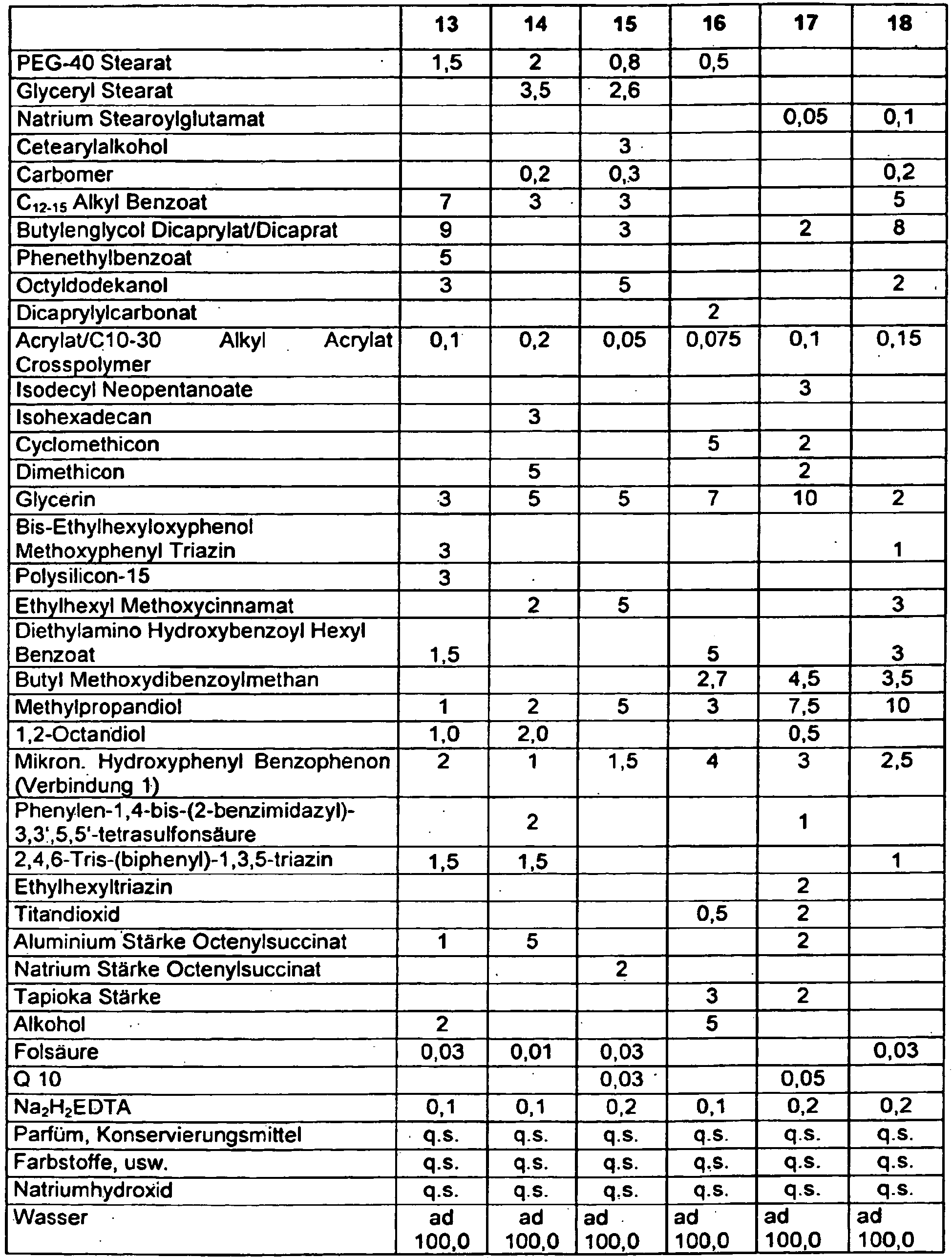

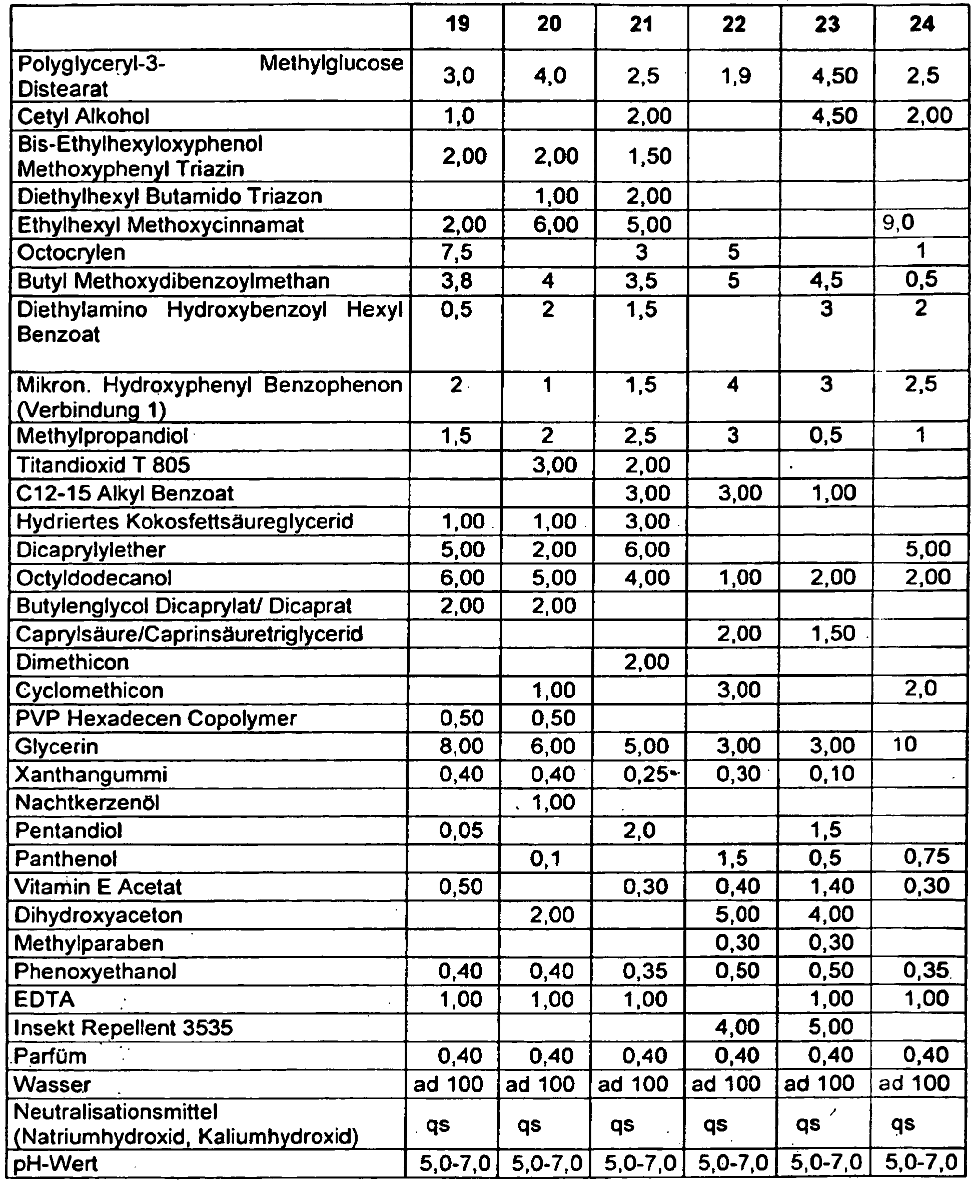

Die Rezepturen können anschließend mit Sauerstoff begast werden

### W/O Emulsion

| **Emulsion** | **A** | **B** | **C** | **D** | **E** | **F** |
|---|---|---|---|---|---|---|
| Polyglyceryl-2 Dipolyhydroxystearat | 3 | 5 | 3 | | | |
| PEG-30 Dipolyhydroxystearat | | | 2 | 3 | 4 | 5 |
| Natrium-Stärke Octenylsuccinat | 0,5 | 0,4 | 0,6 | 0,3 | 0,5 | 1 |
| Glycin | 0,3 | 0,3 | 0,5 | 0,4 | | |
| Alkohol | 2 | 5 | 2 | 5 | 4 | 3 |
| Magnesiumsulfat | 0,2 | 0,3 | 0,3 | 0,4 | 0,5 | 0,2 |
| C₁₂₋₁₅ Alkyl Benzoat | 5 | 3 | | | 5 | |
| Triheptanoin | | 2 | | | | |
| Butylenglycol Dicaprylat/Dicaprat | 5 | | | | 3 | 3 |
| Dicaprylyl Ether | | | | | 2 | |
| Mineralöl | | 4 | | 6 | | 8 |
| Octyldodecanol | 2 | | | | | |
| Dicaprylcaprat | | 2 | | | 2 | 2 |
| Cyclomethicon | 5 | | 5 | 10 | | |
| Dimethicon | | | | 5 | | |
| 1,2-Octandiol | 0,5 | 1 | | | 1,25 | |
| Butylenglycol | 5 | 8 | | | | 3 |
| Glycerin | 3 | 5 | 7 | 10 | 3 | 3 |
| Ethylhexylmethoxycinnamat | 5 | | 7 | | 5 | |
| 2,4,6-Tris-(biphenyl)-1,3,5-triazin | | 0,5 | | | 0,5 | |
| Ethylhexyltriazin | 2 | 3 | 3 | | | 3 |
| Diethylhexylbutamidotriazin | | 1,5 | 1,5 | | | 1,5 |
| Methylpropandiol | 0,5 | 1,5 | 3 | 2 | 0,5 | 0,75 |
| Butyl Methoxydibenzoylmethan | 3,9 | 4 | 3,5 | 2,5 | 4 | 3 |
| Phenylbenzimidazol Sulfonsäure | 0,5 | 2 | 0,5 | 2 | 3 | 2 |
| Mikron. Hydroxyphenyl Benzophenon (Verbindung 1) | 2 | 10 | 1,5 | 4,5 | 3 | 0,5 |
| 2-(4'-Diethylamino-2'-hydoxybenzoyl)-benzoesäurehexylester | | 1,5 | | 2 | | |
| Titandioxid | 1 | | | | 2 | 4 |
| Panthenol | 1 | 0,25 | 2 | | 0,5 | 0,75 |
| Merocyanin | 2 | | 2 | 5 | 1 | |
| Vitamin E Acetat | 0,2 | 0,2 | 0,2 | 0,3 | 0,1 | 0,5 |
| Na₂H₂EDTA | 0,1 | 0,1 | 0,2 | 0,2 | 0,2 | 0,5 |
| Parfüm, Konservierungsmittel | q.s. | q.s. | q.s. | q.s. | q.s. | q.s. |
| Wasser | ad 100,0 | ad 100,0 | ad 100,0 | ad 100,0 | ad 100,0 | ad 100,0 |

### Hydrodispersionen

| | **1** | **2** | **3** | **4** | **5** | **6** |
|---|---|---|---|---|---|---|
| Glyceryl Stearat Citrat | | 0,40 | | | | |
| Sodium Carbomer | | | | | 0,30 | |
| Acrylates/C 10-30 Alkyl Acrylate Crosspolymer | | | 0,30 | 0,40 | 0,10 | 0,10 |
| Ceteareth-20 | | | 1,00 | | | |
| Xanthan Gummi | 0,50 | | | 0,15 | | 0,50 |
| Dimethicon / Vinyl Dimethicon Crosspolymer | | | | 5,00 | | 3,00 |
| UVASorb® K2A | | | | | 3,50 | |
| 2-(4'-Diethylamino-2'-hydoxybenzoyl)-benzoesäurehexylester | 0,25 | | | 0,50 | 2,00 | 1,50 |
| Bis-Ethylhexyloxyphenol Methoxypheny Triazin | | 2,00 | | 0,25 | | |
| Dinatrium Phenyl Dibenzimidazol Tetrasulfonat | 0,75 | | | | | 1,00 |
| Ethylhexyl Methoxycinnamat | | | 7,00 | | 5,00 | 8,00 |
| Diethylhexyl Butamido Triazin | | | 2,00 | 2,00 | | |
| Ethylhexyl Triazin | 4,00 | 3,00 | | | 4,00 | |
| Titandioxid | 0,50 | 2,00 | 1,00 | 2,00 | 3,00 | 1,00 |
| Octocrylen | | 9,5 | 3 | 2 | | |
| Butyl Methoxydibenzoylmethan | 4,2 | | 3,5 | 2 | 4,5 | 1 |
| Methylpropandiol | 0,5 | 2 | 1,5 | 2,5 | 3 | 2 |
| Mikron. Hydroxyphenyl Benzophenon (Verbindung 1) | 2 | 1 | 1,5 | 4 | 3 | 2,5 |
| C₁₂₋₁₅ Alkyl Benzoat | 2,00 | | 2,50 | | | |
| C18-36 Triglycerid Fettsäure | | | 1,00 | | | |
| Butylenglycol Dicaprylat/Dicaprat | 4,00 | | | | 6,00 | |
| Dicaprylyl Carbonat | | 3,00 | | | | |
| Dicaprylylether | | 2,00 | | | | |
| Cyclomethicon | | | | 7,50 | | |
| PVP Hexadecen Copolymer | 0,50 | | 0,50 | | 0,50 | 1,00 |
| Glycerin | 10,00 | 5,00 | 5,00 | | 5,00 | 15,00 |
| Butylenglycol | | 7,00 | | | | |
| 1,2-Hexandiol | 0,50 | | | 1,00 | | 1,50 |
| Vitamin E Acetat | 0,50 | 0,25 | 0,50 | 0,25 | 0,75 | 1,00 |
| Panthenol | 1,50 | 0,50 | | | 0,25 | |
| Trinatrium EDTA | | 1,00 | 1,00 | 0,10 | 0,20 | |
| Ethanol | 3,00 | | 4,00 | 3,50 | | 1,00 |
| Konservierungsmittel | q.s. | q.s. | q.s. | q.s. | q.s. | q.s. |
| Parfüm, Farbstoffe, | q.s. | q.s. | q.s. | q.s. | q.s. | q.s. |
| Wasser | ad 100 | ad 100 | ad 100 | ad 100 | ad 100 | ad 100 |

### Gele

| | **1** | **2** | **3** | **4** | **5** | **6** |
|---|---|---|---|---|---|---|
| Acrylat/Octylacrylamid Copolymer | 1,0 | 1,0 | 1,0 | 1,0 | 1,0 | 1,0 |
| Alkohol Denat. | 50,0 | 62,0 | 59,2 | 70,0 | 70,0 | 69,0 |
| Butylen Glycol Dicaprylat/Dicaprat | | | 9,5 | | | |
| C12-15 Alkyl Benzoat | 5,0 | 10,0 | 5,0 | 5,0 | 9,5 | |
| Phenylbenzoat | 5,0 | | | | | |
| Cocoglycerid | | | | | | 5 |
| Diethylamino Hydroxybenzoyl Hexyl Benzoat | | | 4,5 | 3,5 | | |
| Ethylhexyl Methoxycinnamat | 5 | 9,5 | | 6,5 | 6,5 | 9,5 |
| Ethylhexyl Salicylat | 4,5 | 4,5 | 4,5 | 4,5 | | |
| Homosalat | | | | | | 4,5 |
| Hydroxypropylcellulose | 2 | 0,8 | 1 | 0,8 | 0,5 | 0,8 |
| Octocrylen | 7,5 | 9,5 | 3 | 2 | 2 | 3 |
| Butyl Methoxydibenzoylmethan | 4,2 | 4 | 3,5 | 2 | 4,5 | 3 |
| Methylpropandiol | 0,5 | 2 | 1,5 | 2,5 | 3 | 2 |
| Mikron. Hydroxyphenyl Benzophenon (Verbindung 1) | 2 | 1 | 1,5 | 4 | 3 | 2,5 |
| Ethylhexyltriazin | | | | | 2 | |
| Benzophenone-3 | 3 | | | | | |
| Octyl 5-N,N-diethylamino-2-phenylsulphonyl-2,4-pentadienoat | 1 | 0,5 | 2 | 3 | 1 | 5 |
| Bis-Ethylhexyloxyphenol Methoxyphenyltriazin | | | | | | 1 |
| Vitamin E Acetat | | | | 0,5 | | 0,2 |
| Glycerin | 5 | | 3 | | | |
| Parfüm, Farbstoffe | q.s. | q.s. | q.s. | q.s. | q.s. | q.s. |
| Wasser | ad 100 | ad 100 | ad 100 | ad 100 | ad 100 | ad 100 |

### Spray

| | **1** | **2** | **3** | **4** | **5** | **6** |
|---|---|---|---|---|---|---|
| Acrylat/Octylacrylamid Copolymer | 1 | | | | 1 | 1 |
| VPNA Copolymer | | 1,0 | | 0,5 | | |
| Alkohol Denat. | 46,5 | 60 | 63 | 60 | 45,5 | 46,5 |
| Bis-Ethylhexyloxyphenol Methoxyphenyl Triazin | | | | | 1,0 | |
| Uvasorb® K2A | 0,5 | | 2,0 | | | 3,0 |
| Butyl Methoxydibenzoylmethan | 4,5 | 2 | | 3 | 4,5 | |
| Cyclomethicon | 6,9 | 2 | 5 | 0,5 | 8,0 | 8 |
| Diethylamino Hydroxybenzoyl Hexyl Benzoat | | 2 | 3 | | | 4,5 |
| Ethylhexyl Methoxycinnamat | 9,5 | | | 7 | 6,5 | 9,5 |
| Ethylhexyl Salicylat | 4,5 | 4 | 2 | 4 | 4,5 | 4,5 |
| Homosalat | 9,5 | 5 | 4 | | 9,5 | 6,5 |
| Octocrylen | 9,5 | | 8 | | 9,5 | 9,5 |
| Mikron. Hydroxyphenyl Benzophenon (Verbindung 1) | 0,5 | 1,15 | 1,5 | 4 | 3 | 3,5 |
| Methylpropandiol | 0,5 | 0,75 | 1,5 | 2,5 | 0,75 | 1,0 |
| Merocyanin | | | 2 | | | |
| Butylen Glycol Dicaprylat/Dicaprat | | 9 | | | | |
| C12-15 Alkyl Benzoat | | 2 | | 2 | | |
| Phenylbenzoat | | | 7 | | | |
| Phenyl Trimethicone | 2 | 5 | | | 2 | 2 |
| 1,2-Hexandiol | 0,5 | 1,5 | | 0,75 | | 1,0 |
| Glycerin | 5 | 4 | 5 | 8 | 5 | 5 |
| Vitamin E Acetat | 0,1 | | 0,5 | | | |
| Parfüm, Farbstoffe | q.s. | q.s. | q.s. | q.s. | q.s. | q.s. |
| Wasser | ad 100 | ad 100 | ad 100 | ad 100 | ad 100 | ad 100 |

## Patentansprüche

1. Kosmetische Zubereitung enthaltend
a) 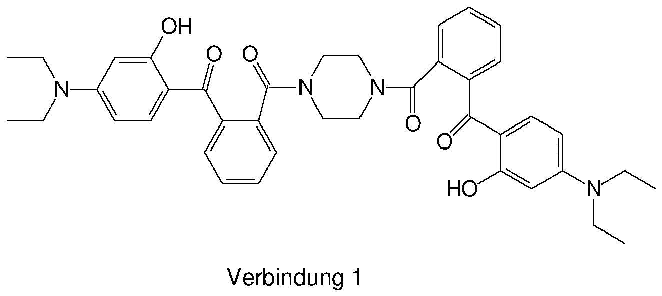
b) ein oder mehrere 1,3-Alkandiol, wobei als 1,3-Alkandiol 2-Methyl-1,3-propandiol eingesetzt wird.

2. Kosmetische Zubereitung nach Anspruch 1, **dadurch gekennzeichnet, dass** die Zubereitung Verbindung 1 in einer Konzentration von 0,25 bis 10 Gewichts-%, bezogen auf das Gesamtgewicht der Zubereitung, enthält.

3. Kosmetische Zubereitung nach einem der vorhergehenden Ansprüche, **dadurch gekennzeichnet, dass** die Zubereitung 1,3-Alkandiole in einer Konzentration von 0,2 bis 5 Gewichts-%, bezogen auf das Gesamtgewicht der Zubereitung, enthält.

4. Kosmetische Zubereitung nach einem der vorhergehenden Ansprüche, **dadurch gekennzeichnet, dass** die Zubereitung einen oder mehrere weitere UV-Filter enthält, gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäureamylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, 4-(tert.-Butyl)-4'-methoxydibenzoylmethan; Homomenthylsalicylat; 2-Ethylhexyl-2-hydroxybenzoat; 2-Ethylhexyl-2-cyano-3,3-diphenylacrylat; Dimethicodiethylbenzalmalonat; 3-(4-(2,2-bis Ethoxycarbonylvinyl)-phenoxy)propenyl)-methoxysiloxan / Dimethylsiloxan - Copolymer; 2,4-Bis-{[4-(2-ethyl-hexyloxy)-2-hydroxy]-phenyl}-6-(4-methoxyphenyl)-1,3,5-triazin (INCI :BisEthylhexyloxyphenol Methoxyphenyl Triazin); Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titiandioxid; Zinkoxid in einer Konzentration von 0,01 bis 40 Gewichts-% bezogen auf das Gesamtgewicht der Zubereitung.

5. Kosmetische Zubereitung nach einem der vorhergehenden Ansprüche, **dadurch gekennzeichnet, dass** die Zubereitung als weitere Inhaltsstoffe eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, Polydocanol, natürliche und/oder synthetische Isoflavonoide, Flavonoide, Kreatin, Kreatinin, Taurin, ß-Alanin, Tocopherylacetat, Harnstoff; Hyaluronsäure; Dihydroxyaceton; 8-Hexadecen-1,16-dicarbonsäure und/oder Licochalcon A enthält.

6. Kosmetische Zubereitung nach einem der vorhergehenden Ansprüche, **dadurch gekennzeichnet, dass** die Zubereitung in Form einer Emulsion, einer Dispersion, eines Puders, eines Gels oder eines Öls vorliegt.

7. Kosmetische Zubereitung nach einem der vorhergehenden Ansprüche, **dadurch gekennzeichnet, dass** die Zubereitung ein oder mehrere Verbindungen gewählt aus der Gruppe der Parabene, Phenoxyethanol, Ethylhexylglycerin, Butylenglycol, Propylenglycol enthält.

8. Kosmetische Zubereitung nach einem der vorhergehenden Ansprüche, **dadurch gekennzeichnet, dass** die Zubereitung als weitere Inhaltsstoffe eine oder mehrere Verbindungen gewählt aus der Gruppe der Verbindungen eine oder mehrere der Verbindungen 2 bis 9 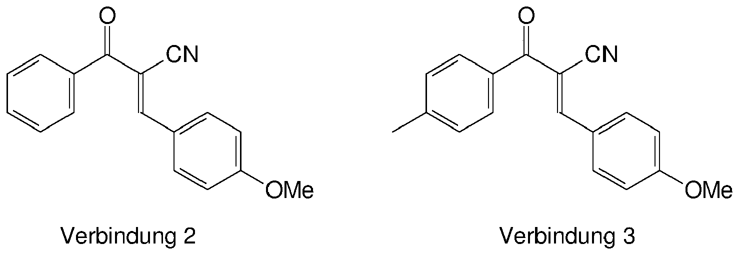 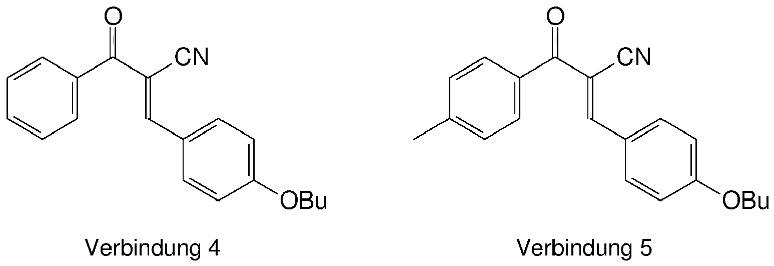 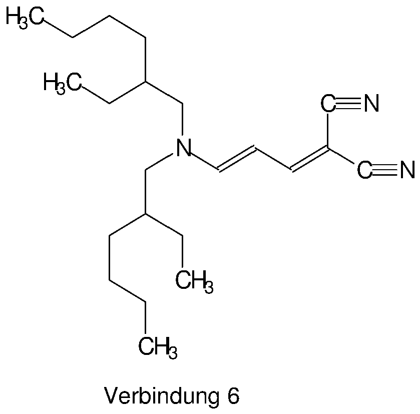 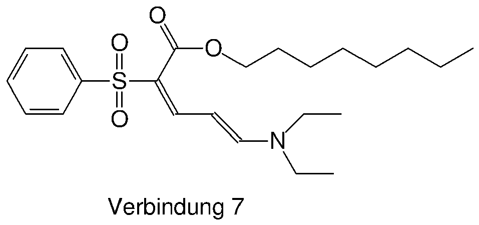 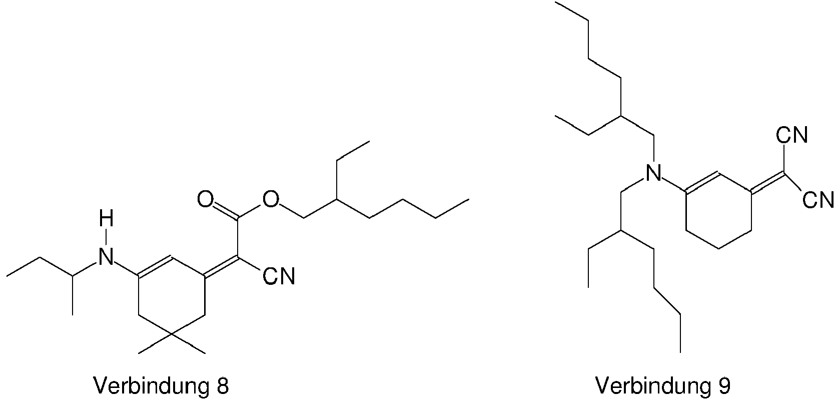 enthält.

9. Kosmetische Zubereitung nach einem der vorhergehenden Ansprüche, **dadurch gekennzeichnet, dass** die Zubereitung in Form einer Emulsion vorliegt.

## Claims

1. Cosmetic preparation comprising
a) 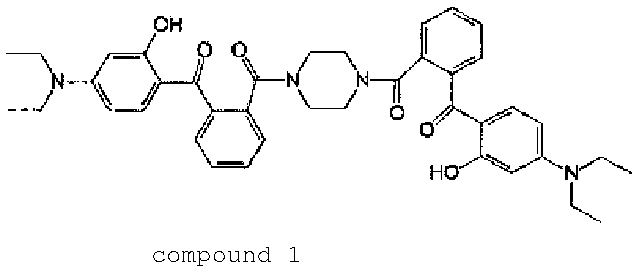
b) one or more 1,3-alkanediol, where the 1,3-alkanediol used is 2-methyl-1,3-propanediol.

2. Cosmetic preparation according to Claim 1, **characterized in that** the preparation comprises compound 1 in a concentration of 0.25 to 10% by weight, based on the total weight of the preparation.

3. Cosmetic preparation according to one of the preceding claims, **characterized in that** the preparation comprises 1,3-alkanediols in a concentration of from 0.2 to 5% by weight, based on the total weight of the preparation.

4. Cosmetic preparation according to one of the preceding claims, **characterized in that** the preparation comprises one or more further UV filters selected from the group of the compounds phenylene-1,4-bis(2-benzimidazyl)-3,3'-5,5'-tetrasulphonic acid salts; 2-phenylbenzimidazole-5-sulphonic acid salts; 1,4-di(2-oxo-10-sulpho-3-bornylidenemethyl)benzene and salts thereof; 4-(2-oxo-3-bornylidenemethyl)benzenesulphonic acid salts; 2-methyl-5-(2-oxo-3-bornylidenemethyl)-sulphonic acid salts; 2,2'-methylenebis(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; terephthalidenedicamphorsulphonic acid; 2-ethylhexyl 4-(dimethylamino)-benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; hexyl 2-(4'-diethylamino-2'-hydroxybenzoyl)benzoate, 4-(tert-butyl)-4'-methoxydibenzoylmethane; homomenthyl salicylate; 2-ethylhexyl 2-hydroxybenzoate; 2-ethylhexyl 2-cyano-3,3-diphenylacrylate; dimethicodiethyl benzalmalonate; 3-(4-(2,2-bis-ethoxycarbonylvinyl)phenoxy)propenyl)methoxysiloxane/dimethylsiloxane copolymer; 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine); dioctylbutylamidotriazone (INCI: Diethylhexyl-Butamidotriazone); 2,4-bis[5-1(dimethylpropyl)benzoxazol-2-yl-(4-phenyl)imino]-6-(2-ethylhexyl)imino-1,3,5-triazine with the CAS No. 288254-16-0; tris(2-ethylhexyl) 4,4',4"-(1,3,5-triazine-2,4,6-triyltriimino)trisbenzoate (also: 2,4,6-tris[anilino(p-carbo-2'-ethyl-1'-hexyloxy)]-1,3,5-triazine (INCI: Ethylhexyl Triazone); 2,4,6-tribiphenyl-4-yl-1,3,5-triazine; merocyanines; titanium dioxide; zinc oxide in a concentration of from 0.01 to 40% by weight, based on the total weight of the preparation.

5. Cosmetic preparation according to one of the preceding claims, **characterized in that** the preparation comprises, as further ingredients, one or more compounds selected from the group of the compounds alpha-lipoic acid, folic acid, phytoene, D-biotin, coenzyme Q10, alpha-glucosylrutin, carnitine, carnosine, polydocanol, natural and/or synthetic isoflavonoids, flavonoids, creatine, creatinine, taurine, β-alanine, tocopheryl acetate, urea; hyaluronic acid; dihydroxyacetone; 8-hexadecene-1,16-dicarboxylic acid and/or licochalcone A.

6. Cosmetic preparation according to one of the preceding claims, **characterized in that** the preparation is present in the form of an emulsion, a dispersion, a powder, a gel or an oil.

7. Cosmetic preparation according to one of the preceding claims, **characterized in that** the preparation comprises one or more compounds selected from the group of parabens, phenoxyethanol, ethylhexylglycerol, butylene glycol, propylene glycol.

8. Cosmetic preparation according to one of the preceding claims, **characterized in that** the preparation comprises, as further ingredients, one or more compounds selected from the group of the compounds
one or more of the compounds 2 to 9

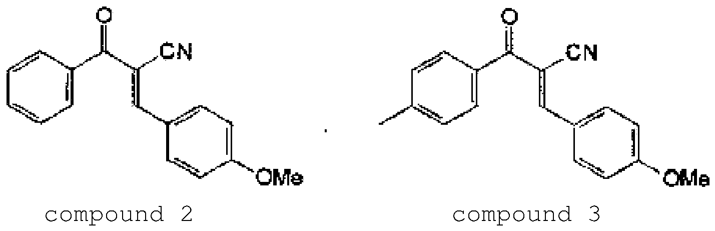

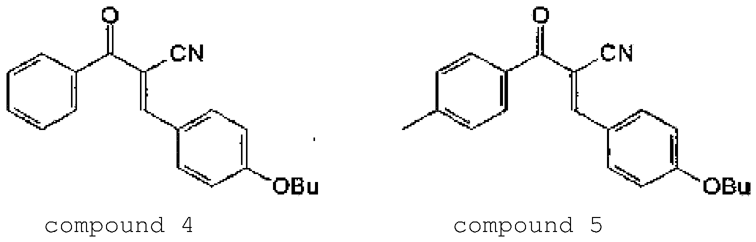

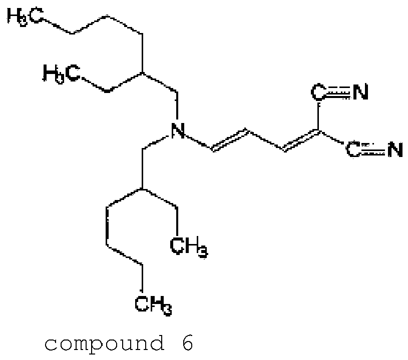

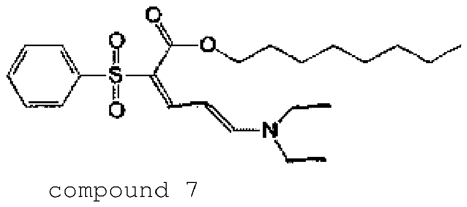

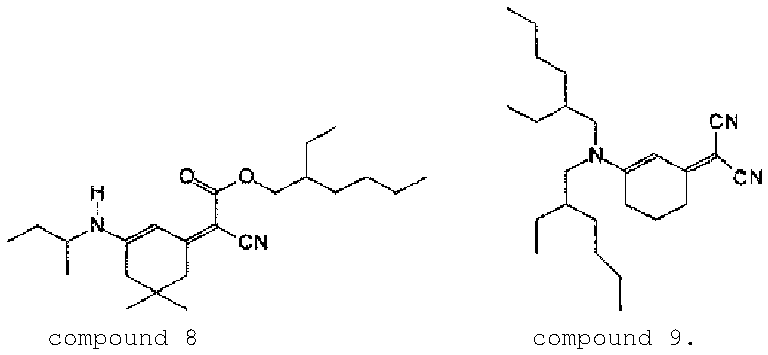

9. Cosmetic preparation according to one of the preceding claims, **characterized in that** the preparation is present in the form of an emulsion.

## Revendications

1. Préparation cosmétique contenant
a) 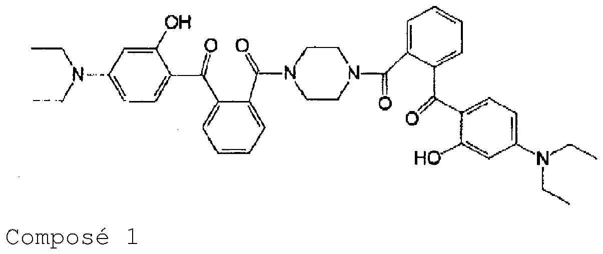
b) un ou plusieurs 1,3-alcanediols, du 2-méthyl-1,3-propanediol étant utilisé comme 1,3-alcanediol.

2. Préparation cosmétique selon la revendication 1, **caractérisée en ce que** la composition contient le composé 1 en une concentration de 0,25 à 10% en poids par rapport au poids total de la composition.

3. Préparation cosmétique selon l'une quelconque des revendications précédentes, **caractérisée en ce que** la composition contient des 1,3-décanediols en une concentration de 0,2 à 5% en poids par rapport au poids total de la composition.

4. Préparation cosmétique selon l'une quelconque des revendications précédentes, **caractérisée en ce que** la composition contient un ou plusieurs autres filtres UV choisis dans le groupe des composés : sels de l'acide phénylène-1,4-bis-(2-benzimidazyl)-3,3'-5,5'-tétrasulfonique ; sels de l'acide 2-phénylbenzimidazole-5-sulfonique ; 1,4-di(2-oxo-10-sulfo-3-bornylidèneméthyl)-benzène et ses sels ; sels de l'acide 4-(2-oxo-3-bornylidèneméthyl)benzènesulfonique ; sels de l'acide 2-méthyl-5-(2-oxo-3-bornylidèneméthyl)sulfonique ; 2,2'-méthylènebis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-(1,3,3,3-tétraméthyl-1-[(triméthylsilyl)oxy]disiloxanyl]propyl]-phénol ; 3-(4-méthylbenzylidène)camphre ; 3-benzylidènecamphre ; salicylate d'éthylhexyle ; acide téréphtalidènedicamphresulfonique ; ester 2-éthylhexylique de l'acide 4-(diméthylamino)-benzoïque ; ester amylique de l'acide 4-(diméthylamino)benzoïque ; ester di(2-éthylhexylique) de l'acide 4-méthoxybenzalmalonique ; ester 2-éthylhexylique de l'acide 4-méthoxycinnamique ; ester isoamylique de l'acide 4-méthoxycinnamique ; 2-hydroxy-4-méthoxybenzophénone, 2-hydroxy-4-méthoxy-4'-méthylbenzophénone ; 2,2'-dihydroxy-4-méthoxybenzophénone ; ester hexylique de l'acide 2-(4'-diéthylamino-2'-hydroxybenzène)-benzoïque, 4-(tert-butyl)-4'-méthoxydibenzoylméthane ; salicylate d'homomenthyle ; 2-hydroxybenzoate de 2-éthylhexyle ; 2-cyano-3,3-diphénylacrylate de 2-éthylhexyle ; benzalmalonate de diméthicodiéthyle ; copolymère de 3-(4-(2,2-bis(éthoxycarbonylvinyl)-phénoxy)propényl)-méthoxysiloxane/diméthylsiloxane ; 2,4-bis-{[4-(2-éthylhexyloxy)2-hydroxy]-phényl}-6-(4-méthoxyphényl)-1,3,5-triazine INCI : bis-Ethylhexyloxyphenol Methoxyphenyl Triazin) ; dioctylbutylamidotriazone (INCI : Diethylhexyl-Butamidotriazone) ; 2,4-bis-[5-1(diméthylpropyl)benzoxazol-2-yl-(4-phényl)-imino]-6-(2-éthylhexyl)-imino-1,3,5-triazine présentant le n° CAS 288254-16-0 ; ester tris(2-éthylhexylique) de l'acide 4,4',4"-(1,3,5-triazine-2,4,6-triyltriimino)-trisbenzoïque (également : 2,4,6-tris-[anilino-(p-carbo-2'-éthyl-1'-hexyloxy)]-1,3,5-triazine (INCI : Ethylhexyl Triazone) ; 2,4,6-tribiphényl-4-yl-1,3,5-triazine ; mérocyanine ; dioxyde de titane ; oxyde de zinc en une concentration de 0,01 à 40% en poids par rapport au poids total de la composition.

5. Préparation cosmétique selon l'une quelconque des revendications précédentes, **caractérisée en ce que** la composition contient, comme autres constituants, un ou plusieurs composés choisis dans le groupe des composés acide alpha-liponique, acide folique, phytoène, D-biotine, coenzyme Q10, alpha-glucosylrutine, carnitine, carnosine, polydocanol, isoflavonoïdes naturels ou synthétiques, flavonoïdes, créatine, créatinine, taurine, ß-alanine, acétate de tocophéryle, urée ; acide hyaluronique ; dihydroxyacétone ; acide 8-hexadécène-1,16-dicarboxylique et/ou licochalcone A.

6. Préparation cosmétique selon l'une quelconque des revendications précédentes, **caractérisée en ce que** la composition se trouve sous forme d'une émulsion, d'une dispersion, d'une poudre, d'un gel ou d'une huile.

7. Préparation cosmétique selon l'une quelconque des revendications précédentes, **caractérisée en ce que** la composition contient un ou plusieurs composés choisis dans le groupe formé par les parabènes, le phénoxyéthanol, l'éthylhexylglycérol, le butylèneglycol, le propylèneglycol.

8. Préparation cosmétique selon l'une quelconque des revendications précédentes, **caractérisée en ce que** la composition contient, comme autres constituants, un ou plusieurs composés choisis dans le groupe des composés
un ou plusieurs des composés 2 à 9

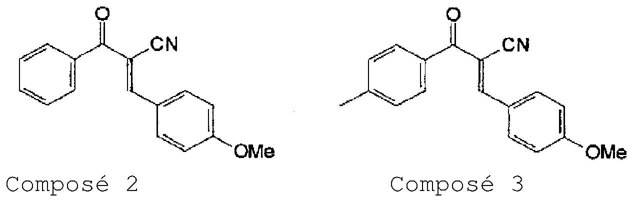

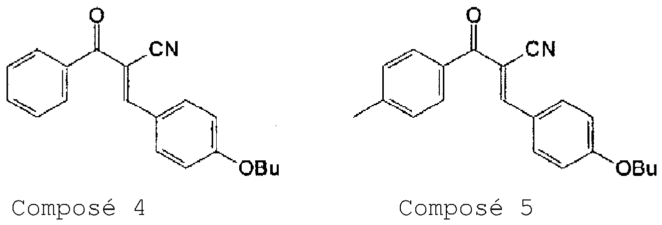

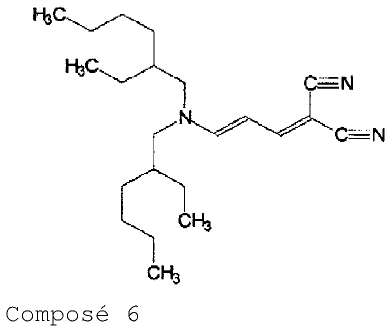

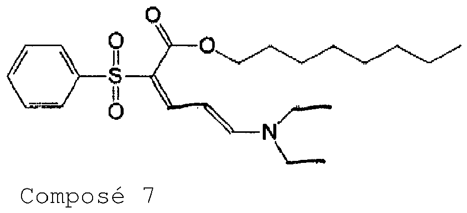

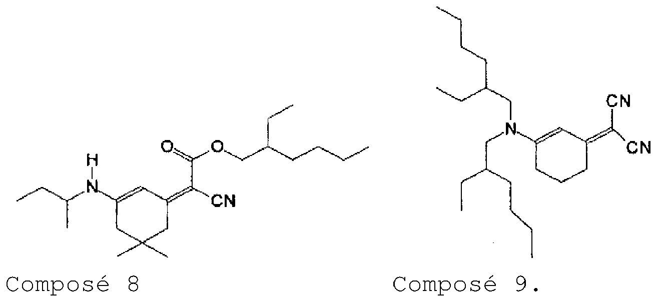

9. Préparation cosmétique selon l'une quelconque des revendications précédentes, **caractérisée en ce que** la préparation se trouve sous forme d'une émulsion.
